# EUROPEAN PATENT SPECIFICATION

(11) **EP 3 654 039 B1**
(45) Date of publication and mention of the grant of the patent: **24.08.2022**
(21) Application number: 18206773.6
(22) Date of filing: 16.11.2018
(51) Int. Cl.: G01N 35/00, G01N 21/59, G01N 35/04, G01N 33/49

(54) **AUTOMATED TUBE HANDLING**
AUTOMATISIERTE REAGENZGLASHANDHABUNG
MANUTENTION AUTOMATISÉE DE TUBES À ESSAIS

(43) Date of publication of application: 20.05.2020
(73) Proprietor: Roche Diagnostics GmbH, 68305 Mannheim (DE); F. Hoffmann-La Roche AG, 4070 Basel (CH)
(72) Inventor: ENCK, Robert, 71332 Waiblingen (DE)
(74) Representative: Lohe, Hans-Jörg

(56) References cited:
- WO-A1-2015/056649
- US-A1- 2016 018 427
- US-A1- 2017 185 815

## Description

### Technical field

Embodiments of the invention refer to a method to identify sample tubes in a diagnostic laboratory and to a diagnostic laboratory system which identifies sample tubes.

### Related art

Diagnostics laboratories are highly automated today. A variety of tubes with different biological samples are processed to get the relevant medical parameters.

Usually in a first step some kind of tube identification device is attached to the tube. The tube identification device such as a RFID-tag, printed bar code or similar can be read out by a respective tube identification reader. These are well known in the art. Based on the identification of the tube the different stations of the automated diagnostic lab can perform the respective task for the specific tube. For instance, in a so called pre-analytic step the tube needs to be centrifuged and maybe sorted to specific racks collecting samples which needs to be analyzed in a specific analyzer.

In addition, in the pre-analytic phase the tube is recognized decapped, if necessary and aliquots are taken into secondary tubes. For these steps the tube type and the liquid level are detected.

US 2016/0266157 discloses an analyte testing automation system, a biological sample check module, and a biological sample check method. The type of blood collection tube inserted into this system is automatically recognized.

US 2016/0018427 concerns a method and system for tube inspection and liquid level detection. Container identification data from a container inspection unit that analyzes a container containing a liquid is combined with liquid level detection raw data from a liquid level detection unit.

### Summary

An object of the invention is to provide for an efficient and reliable processing of sample tubes.

This is realized by a method according to claim 1 and by a diagnostic laboratory system according to claim 6.

A further aspect of the inventive method is that the tube type is determined by one or more of its cap geometry, its cap color, and its tube geometry.

Another aspect of the invention is a method wherein the tube type recognition device is a camera and a control unit of the camera determines the cap geometry and cap color by image processing methods.

An additional aspect of the inventive method is that the tube consistence unit is a laser liquid level detection unit determining the consistence of the tube by scanning the tube.

A further aspect of the inventive method is that the tube identification reader is a barcode reader reading the barcode attached to the tube.

A further aspect of the inventive diagnostics laboratory automation system is that the tube identification device reader is part of a pre-analytic device.

Another aspect of the invention is a diagnostic laboratory automation system wherein the diagnostics laboratory automation system comprises a central automation logic unit adapted to run a laboratory IT system, wherein the control device is comprised in the central automation logic unit.

However, other embodiments are feasible which relates to a combination of features disclosed herewith and fall within the scope of the appended claims.

### Short description of the Figures

Further optional features and embodiments of the invention will be disclosed in more detail in the subsequent description of preferred embodiments, preferably in conjunction with the dependent claims. Therein, the respective optional features may be realized in an isolated fashion as well as in any arbitrary feasible combination, as the skilled person will realize. The scope of the invention is not restricted by the preferred embodiments, but by the appended claims. The embodiments are schematically depicted in the Figures. Therein, identical reference numbers in these Figures refer to identical or functionally comparable elements.

In the Figures:
Fig. 1 is a schematic view of parts of a diagnostic laboratory automation system;
Fig. 2 shows a block diagram of a simplified diagnostic laboratory automation system; and
Fig. 3 shows a block diagram of a method to handle sample tubes.

### Detailed description of the embodiments

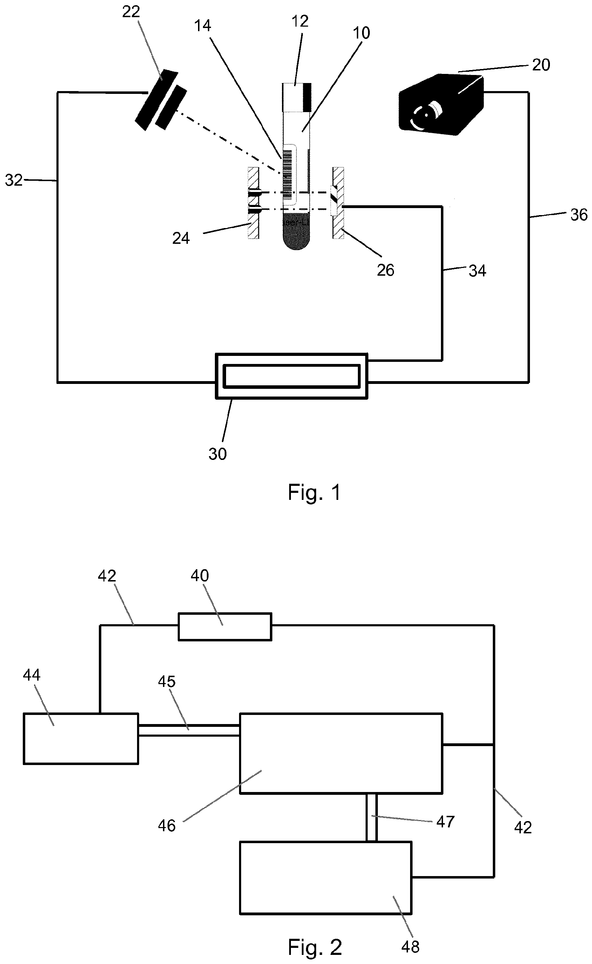

Figure 1 shows a schematic view of parts of a diagnostic laboratory automation system. A tube 10 is usually transported with a not shown transport device through a pre-analytic system or device. The tube 10 comprises usually a cap 12 when it enters the diagnostic laboratory. In the embodiment shown in Fig. 1 the tube 10 comprises a bar code 14 attached to the outer surface of the tube. Other identification devices such as 2D bar code or wireless identification tags such as RFID chips can also be used.

The bar code is read by a bar code reader 22 to identify the sample tube type. The sample tube type related to the bar code is transmitted to a control device 30.

In a next station or place in the pre-analytic system the tube is investigated with a laser liquid level detection system (LLLD) 24, 26. The LLLD system comprises a light emitting part 24 and a light detecting part 26. The tube is scanned with laser light and the detected part of the light which passes the tube is dependent on the content of the tube. Such a system is for example described in WO 2015/049298 herewith incorporated by reference.

With a LLLD system 24, 26 the consistence of the tube is determined, hence whether there exist several separated phases in the content of tube which usually differs by different densities which can be separated by a centrifuge device. The information of the consistence of the tube is also send to the control device 30. For instance, in a blood sample usually two layers exist: in the bottom of the tube a blood clot and above the serum sometimes separated by a separation gel, depending on the kind of tube used.

In a further station or part of the pre-analytic system images of the tube 10 are made with a camera system 20. With image processing means the camera system 20 is calibrated and the tube images are used to identify the geometry of the tube and if applicable of the tube cap as well as the color of the cap and the color of the sample inside the tube.

Image processing methods are used to identify or classify the tube depending on the color of the cap and the geometry of tube and cap. Furthermore, the color of the sample is used to automatically identify or classify the kind of sample.

The image processing methods are realized either in the camera system 20 or at the control device 30 which receives all the information obtained of the tube. Other devices capable of image processing at any appropriate place in the laboratory or via wireless connection to any kind of data processing system locally or in a cloud application are possible, too.

The schematic view of Fig. 1 is simplified in such that it looks like that the bar code- reader 22 the camera 20 and the LLLD 24,26 are inspecting the tube at the same point in space in the diagnostic laboratory automation system. This is usually not the case and these devices are installed at different areas in the laboratory or in the pre-analytic system with the respective environment to support the respective functionality e.g. by shielding the used laser beams, provide a background for better imaging etc. Nevertheless, the operations are usually centralized in a pre-analytic instrument or in an entry area of tubes of the laboratory to guarantee a meaningful sequential and coherent processing of the tubes in the lab.

Fig. 2 shows a schematic block diagram of a diagnostic laboratory automation system.

A pre-analytic device 44 receives the sample tubes. These are identified, classified, decapped, aliquot into secondary tubes and maybe recapped and the sample tube and/or its aliquots are put on a transport device 45. The transport device 45 transports the tubes to an analyzer 46 to analyses the sample in the tubes. Afterwards the tubes can be transported to a post analytic system 48 by a transport device 47. In the post-analytic device 48 the tubes are stored in controlled environmental conditions and if necessary resend to the analyzer 46 or any other not shown analyzer or casted away in the appropriate way.

A lab control unit 40 is connected to any device via a lab bus 42 to control the required way and analysis of the samples in the tubes. If the lab control unit 40 receives the data of the pre-analytic system 44 as described above with reference to Fig. 1 the lab control unit 40 can adjust the correct sample tube type to directly send the tubes to the necessary analyzer 46 or at least to order at the analyzer 46 the necessary and appropriate analysis.

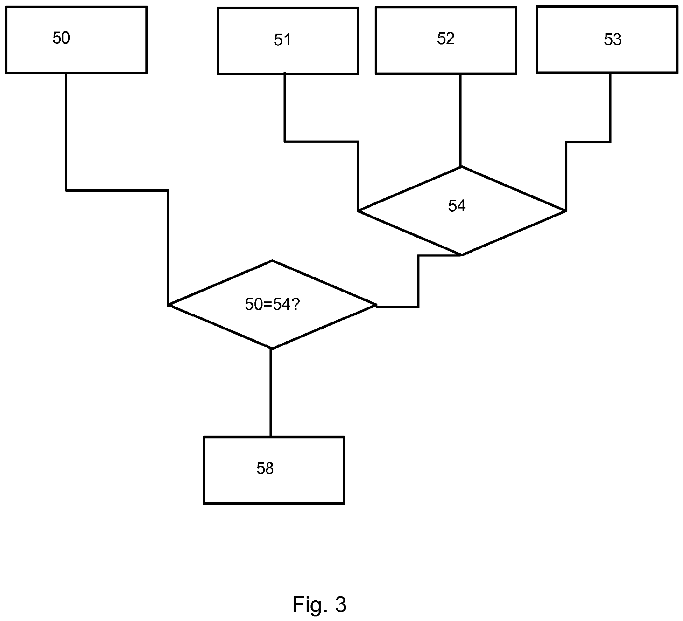

Fig. 3 is a block diagram of the method to handle sample tubes in the diagnostic laboratory. The tube identification device reader identifies the sample tube type 50. The tube type recognition device identifies the type of tube 51. The sample color determination unit determines the color of the sample 52. The tube type recognition device and the sample color determination unit are both realized in the embodiment shown in Fig. 1 by one device: the camera system 20. This does not need to be the case. The tube consistence unit determines the consistency of the sample 53.

Out of the type of the tube 51, the color of the sample 52 and the consistency of the sample 53 the method determines a construed sample tube type 54. This can be realized by a decision table.

For instance, if the type of tube 51 is related to a kind of tube used for urine, the color of the sample is yellow and the consistency of the sample is uniform, then the construed sample tube type will be urine.

In another case if the type of tube is related to a kind used for blood samples, the color is yellow due to a lipaemic sample and the consistency identifies three distinguished layer the construed sample tube type will be blood, centrifuged.

So the construed sample tube type classifies in this example into urine, blood non centrifuged and blood centrifuged.

The following table summarizes the classifications which are at least possible:

| Sample type\class | type of tube | Color of sample | consistency |
|---|---|---|---|
| Urine | urine | yellow, clear | one phase |
| Urine, hemolytic | urine | light red | one phase |
| Urine lipaemic | urine | white-yellow, opaque | one phase |
| Blood with separation gel | blood | red and white clear | two phases |
| Full blood | blood | red | one phase |
| Blood sedimented | blood | clear and dark red | two phases |
| Blood with gel centrifuged | blood | dark red and white and clear | three phases |

Further classification can be implemented depending on the kind of biological liquids used in the laboratory. For example, the last class in the table can be separated in serum icteric, serum hemolytic and serum lipaemic.

It is also possible to consider measurement errors or overlapping classification classes in the classes. Also it could happen that a sample is in the "wrong" or not expected tube. Than the color of the sample and the consistency would contradict the type of tube classification. In these cases, the determination of the construed sample tube type will have a confidence level, e.g. of 2/3 if two of three classes identifies the sample type. This can be used to only rely to the determination if a given confidence level is reached by the classification.

If the confidence level cannot be reached an error message can be generated and the respective tube can be send to an error handling area in the laboratory automation system.

The color identification can be realized by color values determined from the camera which needs to be calibrated for a specific color space. This will allow to better distinguish the different sample types

In a further step of the method the construed sample tube type 54 is compared with the sample tube type 50. If they are equal the sample tube type 50 will be used as the final result 58 to decide on the further processing of the sample.

If the construed sample tube type 54 and the sample tube type are different, then the construed sample tube type 54 is used as the final result 58 of the method to decide on further processing of the sample.

If the construed sample tube type 54 cannot be determined due to inconsistencies in the type of tube 50, the color of the sample 52 and the consistency of the sample 53 the sample will be send to an error output station in laboratory for further investigations on the sample tube.

## Claims

1. A method to handle sample tubes in a diagnostic laboratory automation system comprising a control device and a tube analyzing device, wherein the tube analyzing device comprises a tube identification device reader (22), a tube type recognition unit (20), a sample color determination unit (20), and a tube consistence unit (24, 26),
wherein the tube identification device reader reads a tube identification device of a sample tube to determine the sample tube type,
wherein the tube type recognition device identifies the type of tube,
wherein the sample color determination unit determines the color of the sample in the sample tube,
wherein the tube consistence unit determines the consistency of the sample in the tube, consisting of determining whether there exist several separated phases in the content of tube,
wherein the sample tube type, the tube type, the color of the sample, and the consistency of the sample are sent to the control device,
**characterized in that** the control device determines a construed sample tube type construed out of all of the information of the type of tube, the color of the sample, and the consistency of the sample in the tube, and **in that** the control device determines a confidence level of the construed sample tube type out of all of the information of the type of tube, the color of the sample, and the consistency of the sample in the tube, and only changes the used sample tube type from the sample tube type to the construed sample tube type if the confidence level is above or equal a predetermined confidence level.

2. A method according to one of the preceding claims wherein the tube type is determined by one or more of its cap geometry, its cap color, and its tube geometry.

3. A method according to the preceding claim wherein the tube type recognition device is a camera and a control unit of the camera determines the cap geometry and cap color by image processing methods.

4. A method according to one of the preceding claims wherein the tube consistence unit is a laser liquid level detection unit determining the consistence of the tube by scanning the tube.

5. A method according to one of the preceding claims wherein the tube identification reader is a barcode reader reading the barcode attached to the tube.

6. A diagnostics laboratory automation system comprising a control device and a tube analyzing device, wherein the tube analyzing device comprises a tube identification device reader, a tube type recognition unit, a sample color determination unit, and a tube consistence unit, **characterized in that** the system is adapted to perform the method according to one of the preceding claims.

7. A diagnostic laboratory automation system according to claim 6, wherein the tube identification device reader is part of a pre-analytic device.

8. A diagnostic laboratory automation system according to one of the claims 6 to 7 wherein the diagnostics laboratory automation system comprises a central automation logic unit adapted to run a laboratory IT system, wherein the control device is comprised in the central automation logic unit.

## Patentansprüche

1. Verfahren zum Handhaben von Probenröhrchen in einem Labordiagnoseautomatisierungssystem, umfassend eine Steuervorrichtung und eine Röhrchenanalysevorrichtung,
wobei die Röhrchenanalysevorrichtung ein Lesegerät für eine Röhrchenidentifikationsvorrichtung (22), eine Röhrchentyperkennungseinheit (20), eine Probenfarbbestimmungseinheit (20) und eine Röhrchenkonsistenzeinheit (24, 26) umfasst,
wobei das Lesegerät für eine Röhrchenidentifikationsvorrichtung eine Röhrchenidentifikationsvorrichtung eines Probenröhrchens liest, um den Probenröhrchentyp zu bestimmen,
wobei die Röhrchentyperkennungsvorrichtung den Typ des Röhrchens identifiziert, wobei die Probenfarbbestimmungseinheit die Farbe der Probe in dem Probenröhrchen bestimmt,
wobei die Röhrchenkonsistenzeinheit die Konsistenz der Probe in dem Röhrchen bestimmt, was aus dem Bestimmen, ob mehrere getrennte Phasen im Inhalt des Röhrchens existieren, besteht,
wobei der Probenröhrchentyp, der Röhrchentyp, die Farbe der Probe und die Konsistenz der Probe an die Steuervorrichtung gesendet werden, **dadurch gekennzeichnet, dass** die Steuervorrichtung einen konstruierten Probenröhrchentyp bestimmt, der aus allen Informationen zum Typ des Röhrchens, der Farbe der Probe und der Konsistenz der Probe in dem Röhrchen konstruiert ist, und dass die Steuervorrichtung ein Vertrauensniveau des konstruierten Probenröhrchentyps aus allen Informationen zum Typ des Röhrchens, der Farbe der Probe und der Konsistenz der Probe in dem Röhrchen bestimmt und lediglich den verwendeten Probenröhrchentyp vom Probenröhrchentyp in den konstruierten Probenröhrchentyp ändert, wenn das Vertrauensniveau über einem vorbestimmten Vertrauensniveau liegt oder diesem gleicht.

2. Verfahren nach einem der vorstehenden Ansprüche, wobei der Röhrchentyp durch eines oder mehrere von seiner Kappengeometrie, seiner Kappenfarbe und seiner Röhrchengeometrie bestimmt wird.

3. Verfahren nach dem vorstehenden Anspruch, wobei die Röhrchentyperkennungsvorrichtung eine Kamera ist und eine Steuereinheit der Kamera die Kappengeometrie und Kappenfarbe mithilfe von Bildverarbeitungsverfahren bestimmt.

4. Verfahren nach einem der vorstehenden Ansprüche, wobei die Röhrchenkonsistenzeinheit eine Laserflüssigkeitspegelerfassungseinheit ist, die die Konsistenz des Röhrchens durch Abtasten des Röhrchens bestimmt.

5. Verfahren nach einem der vorstehenden Ansprüche, wobei das Röhrchenidentifikationslesegerät ein Barcode-Lesegerät ist, das den an dem Röhrchen befestigten Barcode liest.

6. Labordiagnoseautomatisierungssystem, umfassend eine Steuervorrichtung und eine Röhrchenanalysevorrichtung, wobei die Röhrchenanalysevorrichtung ein Lesegerät für eine Röhrchenidentifikationsvorrichtung, eine Röhrchentyperkennungseinheit, eine Probenfarbbestimmungseinheit und eine Röhrchenkonsistenzeinheit umfasst, **dadurch gekennzeichnet, dass** das System dafür ausgelegt ist, das Verfahren nach einem der vorstehenden Ansprüche auszuführen.

7. Labordiagnoseautomatisierungssystem nach Anspruch 6, wobei das Lesegerät für eine Röhrchenidentifikationsvorrichtung Teil einer Voranalysevorrichtung ist.

8. Labordiagnoseautomatisierungssystem nach einem der Ansprüche 6 bis 7, wobei das Labordiagnoseautomatisierungssystem eine zentrale Automatisierungslogikeinheit umfasst, die dafür ausgelegt ist, ein Labor-IT-System ausführen zu lassen, wobei die Steuervorrichtung in der zentralen Automatisierungslogikeinheit enthalten ist.

## Revendications

1. Procédé de manipulation de tubes à échantillon dans un système d'automatisation de laboratoire de diagnostic comprenant un dispositif de commande et un dispositif d'analyse de tubes,
dans lequel le dispositif d'analyse de tubes comprend un lecteur de dispositif d'identification de tube (22), une unité de reconnaissance de type de tube (20), une unité de détermination de couleur d'échantillon (20) et une unité de consistance de tube (24, 26),
dans lequel le lecteur de dispositif d'identification de tube lit un dispositif d'identification de tube d'un tube à échantillon pour déterminer le type de tube à échantillon,
dans lequel le dispositif de reconnaissance de type de tube identifie le type de tube, dans lequel l'unité de détermination de couleur d'échantillon détermine la couleur de l'échantillon dans le tube à échantillon,
dans lequel l'unité de consistance de tube détermine la consistance de l'échantillon dans le tube, consistant à déterminer s'il existe plusieurs phases séparées dans le contenu du tube,
dans lequel le type de tube à échantillon, le type de tube, la couleur de l'échantillon et la consistance de l'échantillon sont envoyés au dispositif de commande, **caractérisé en ce que** le dispositif de commande détermine un type de tube à échantillon interprété qui est interprété à partir de l'ensemble des informations du type de tube, de la couleur de l'échantillon et de la consistance de l'échantillon dans le tube, et **en ce que** le dispositif de commande détermine un niveau de confiance du type de tube à échantillon interprété à partir de l'ensemble des informations du type de tube, de la couleur de l'échantillon et de la consistance de l'échantillon dans le tube, et modifie uniquement le type de tube à échantillon utilisé du type de tube à échantillon jusqu'au type de tube à échantillon interprété si le niveau de confiance est supérieur ou égal à un niveau de confiance prédéterminé.

2. Procédé selon l'une des revendications précédentes dans lequel le type de tube est déterminé par une ou plusieurs parmi sa géométrie de capuchon, sa couleur de capuchon et sa géométrie de tube.

3. Procédé selon la revendication précédente dans lequel le dispositif de reconnaissance de type de tube est une caméra et une unité de commande de la caméra détermine la géométrie du capuchon et la couleur du capuchon par des méthodes de traitement d'images.

4. Procédé selon l'une des revendications précédentes dans lequel l'unité de consistance de tube est une unité de détection laser de niveau de liquide déterminant la consistance du tube par balayage du tube.

5. Procédé selon l'une des revendications précédentes dans lequel le lecteur d'identification de tube est un lecteur de codes-barres lisant le code-barres fixé au tube.

6. Système d'automatisation de laboratoire de diagnostic comprenant un dispositif de commande et un dispositif d'analyse de tubes, dans lequel le dispositif d'analyse de tubes comprend un lecteur de dispositif d'identification de tube, une unité de reconnaissance de type de tube, une unité de détermination de couleur d'échantillon et une unité de consistance de tube, **caractérisé en ce que** le système est conçu pour réaliser le procédé selon l'une des revendications précédentes.

7. Système d'automatisation de laboratoire de diagnostic selon la revendication 6, dans lequel le lecteur de dispositif d'identification de tube fait partie d'un dispositif pré-analytique.

8. Système d'automatisation de laboratoire de diagnostic selon l'une des revendications 6 à 7 dans lequel le système d'automatisation de laboratoire de diagnostic comprend une unité logique d'automatisation centrale conçue pour exécuter un système IT de laboratoire, dans lequel le dispositif de commande est compris dans l'unité logique d'automatisation centrale.
